# EUROPÄISCHE PATENTSCHRIFT

(11) **EP 0 643 031 B1**
(45) Veröffentlichungstag und Bekanntmachung des Hinweises auf die Patenterteilung: **05.11.1997**
(21) Anmeldenummer: 94113600.4
(22) Anmeldetag: 31.08.1994
(51) Int. Cl.: C07C 29/16, C07C 29/17, C07C 31/125, C07C 45/50, C07C 45/49, C07C 47/02, C07C 47/12, C07C 47/21

(54) **Verfahren zur Hydroformylierung von Butadien-1.3**
Method of hydroformylating 1,3-butadiene
Procédé d'hydroformylation de butadiène-1.3

(30) Priorität: 09.09.1993 DE 4330489
(43) Veröffentlichungstag der Anmeldung: 15.03.1995
(73) Patentinhaber: HOECHST AKTIENGESELLSCHAFT, 65926 Frankfurt am Main (DE)
(72) Erfinder: Fell, Bernhard, Prof.Dr., D-52074 Aachen (DE); Hermanns, Peter, D-52078 Aachen (DE)

(56) Entgegenhaltungen:
- EP-A- 0 033 554
- EP-A- 0 366 089
- DE-A- 2 627 354
- US-A- 4 426 542

## Beschreibung

Die Erfindung betrifft ein Verfahren zur Hydroformylierung von Butadien-1,3 mit dem Ziel, Vorstufen des 2-Propylheptanols sowie weitere Wertprodukte zu erhalten.

Primäre, einwertige Alkohole mit 8 bis 10 Kohlenstoffatomen werden, mit Phthalsäure verestert, in großem Umfang als Weichmacher, insbesondere für Polyvinylchlorid, eingesetzt. Alkohole mit kürzerer Kohlenstoffkette ergeben Weichmacher mit guter Gelierkraft, nachteilig ist jedoch ihre höhere Flüchtigkeit. Längerkettige Alkohole führen als Phthalsäureester zu Weichmachern, die langsam gelieren und schlechtere Kältebeständigkeit besitzen.

Die Eigenschaften der Phthalsäureester-Weichmacher werden außer durch die Länge auch durch die Verzweigung der Kohlenstoffkette im Alkoholmolekül geprägt. Wenig verzweigte Alkohole ergeben Esterweichmacher, die wegen ihrer hohen Kälteflexibilität besonders geschätzt sind. Weitgehend lineare Alkohole mit 8 bis 10 Kohlenstoffatomen im Molekül gewinnen daher als Alkoholkomponente zunehmend an Bedeutung. Voraussetzung für ihren Einsatz ist, daß sie in großen Mengen und kostengünstig zur Verfügung stehen. Gegenwärtig ist das 2-Ethylhexanol die wichtigste Alkoholkomponente für Phthalsäureester. Man ist jedoch bestrebt, weitere Rohstoffe als Basis für die Herstellung von Alkoholen mit den genannten Eigenschaften zu erschließen.

Nach der DE-C-2 855 421 werden als Weichmacher Phthalate von C₉-Alkoholen eingesetzt, die durch Oxoreaktion von C₈-Olefinen, Hydrierung des Reaktionsproduktes und Veresterung der Alkohole mit Phthalsäureanhydrid erhalten werden. An die Ausgangsolefine werden bestimmte Forderungen gestellt. Sie sollen zu 3 bis 20 Gew.-% aus Verbindungen mit einem Isobutanskelett und zu weniger als 3 Gew.-% aus Verbindungen mit einem quaternären Kohlenstoffatom bestehen. Mehr als 90 Gew.-% der Gesamtmenge der Olefine sollen als n-Octene, Monomethylheptene und Dimethylhexene vorliegen. Schließlich soll das Gewichtsverhältnis der Gesamtmenge der n-Octene und Monomethylheptene zu den Dimethylhexene mehr als 0,8 betragen.

Phthalsäureester auf Basis von C₁₀-Alkoholen sind Gegenstand der europäischen Patentanmeldung 03 66 089. Die C₁₀-Alkohole werden in Form eines Gemisches eingesetzt, das man durch Hydroformylierung einer Butenfraktion, Aldolkondensation des erhaltenen Aldehydgemisches und anschließende Hydrierung gewinnt.

Ein anderer Weg zur Gewinnung von Didecylphthalat-Gemischen ist in der europäischen Patentanmeldung 04 24 767 beschrieben. Die Herstellung der Ester erfolgt nach einem mehrstufigen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches zu einem Gemisch aus Decanolen.

Die bekannten Verfahren erfüllen noch nicht alle Anforderungen, die in wirtschaftlicher und technischer Hinsicht an einen im technischen Maßstab ausgeübten Prozeß gestellt werden, sei es, daß die Ausgangsstoffe nicht in ausreichender Menge und/oder nicht kostengünstig zur Verfügung stehen oder daß die Umwandlung der Ausgangsstoffe in die Alkohole an zu aufwendige Prozesse gebunden ist.

Es bestand daher die Aufgabe, ein Verfahren zu entwickeln, das, von preiswerten Rohstoffen ausgehend, einen technisch einfachen Zugang zu den gewünschten Alkoholen und gegebenenfalls weiteren Wertstoffen eröffnet.

Die Erfindung besteht in einem Verfahren zur Hydroformylierung von Butadien-1,3 bei Temperaturen von 60 bis 150°C und Drücken von 1 bis 20 MPa in Gegenwart einer wäßrigen Lösung als Katalysator, die mindestens eine wasserlösliche Rhodiumverbindung und mindestens ein wasserlösliches, organisches Phosphin enthält. Es ist dadurch gekennzeichnet, daß die Umsetzung des Butadiens bei einem pH-Wert in der wäßrigen Lösung von 8,0 bis 11,0 erfolgt.

Das Ausgangsolefin Butadien-1,3 fällt bei der Herstellung von Ethylen durch thermische Spaltung von Leichtbenzin und höheren Kohlenwasserstoffen zwangsweise in erheblichen Mengen an. Man isoliert es aus den C₄-Crackschnitten des Pyrolyseproduktes, z.B. durch Flüssig-Flüssig-Extraktion mit einem selektiven Lösungsmittel wie Acetonitril, Dimethylformamid oder N-Methylpyrrolidon. Es ist überdies durch Dehydrierung von Butan oder Buten leicht zugänglich.

Zur Hydroformylierung setzt man Butadien-1,3 in der heute handelsüblichen Form ein, d.h. in einer Reinheit von mindestens 99,5 Gew.-%. Die Hydroformylierung des konjugierten Diolefins ist schon mehrfach durchgeführt worden. Sie läuft sowohl unter der Einwirkung von Kobalt- als auch von Rhodiumkatalysatoren ab. Die durch Kobalt katalysierte Umsetzung wird z.B. von Adkins und Williams (J.Org.Chem. 17, 980 (1952)) beschrieben. Sie führt in mäßiger Ausbeute zu einem Gemisch aus n- und i-Valeraldehyd im Molverhältnis 1 : 1.

Günstigere Aldehydausbeuten erzielt man bei der Hydroformylierung von Butadien-1,3 in Gegenwart von Rhodiumkatalysatoren. Bewährt haben sich Komplexverbindungen des Rhodiums mit einem mehrzähnigen Liganden, der dreiwertige Phosphoratome enthält. Ein auf derartigen Katalysatoren beruhendes Verfahren ist z.B. in der EP-B-33 554 beschrieben.

Im Rahmen der vorliegenden Erfindung wird die Hydroformylierung des Butadiens-1,3 in einem heterogenen Zweiphasensystem durchgeführt. Die Grundlagen dieses Prozesses sind z.B. in der DE-C-26 27 354 beschrieben. Der Prozeß ist charakterisiert durch das Vorliegen einer organischen Phase, die das Ausgangsolefin und das Reaktionsprodukt enthält und einer wäßrige Phase, in der der Katalysator gelöst ist. Als Katalysatoren finden wasserlösliche Rhodiumkomplexverbindungen mit wasserlöslichen, organischen Phosphinen als Liganden Anwendung. Zu den wasserlöslichen Phosphinen zählen insbesondere Trialkylphosphine, Triarylphosphine, Tri(alkyl,aryl)-phosphine und Alkylendiphosphine und Aryldiphosphine, die mindestens einen durch eine Sulfonsäuregruppe oder eine Carboxylgruppe substituierten organischen Rest enthalten. Ihre Herstellung ist bekannt, vgl. z.B. DE-C-26 27 354 und DD-A-259 194. Besonders geeignet sind Triphenylphosphinmono-, -di- und -trisulfonate, die als individuelle Verbindungen oder als Gemisch aus 2 oder 3 Sulfonatkomponenten eingesetzt werden können. Überraschenderweise gelingt es nach der neuen Arbeitsweise, durch Einhalten eines pH-Wertes von 8,0 bis 11,0 in der wäßrigen Phase während der Reaktion die Selektivität der Umsetzung hinsichtlich der Bildung von C₅-Aldehyden und Folgeprodukten der C₅-Aldehyde beträchtlich zu steigern. Zu den Folgeprodukten der C₅-Aldehyde, die im Verlauf der Hydroformylierung auftreten, gehören als intermolekulare Kondensationsprodukte ungesättigte C₁₀-Aldehyde, die zu 2-Propylheptanol hydriert werden können. Ein weiteres Folgeprodukt ist das intramolekulare Kondensationsprodukt von Hexandial-1,6, das nach Wasserabspaltung und Wasserstoffanlagerung Cyclopentanaldehyd und nach Hydrierung Cyclopentanmethanol, ein wertvolles Zwischenprodukt für organische Synthesen, ergibt. Besonders bewährt hat es sich, bei pH-Werten im Bereich von 8,5 bis 9,5 zu arbeiten.

Die das erfindungsgemäße Verfahren kennzeichnenden pH-Werte beziehen sich auf die wäßrige Phase während der Reaktion, also im wesentlichen auf die Lösung des aus Rhodium und wasserlöslichem organischen Phosphin bestehenden Katalysators. Die Bestimmung des pH-Wertes erfolgt in bekannter Weise, z.B. mit Hilfe einer Wasserstoffelektrode.

Der pH-Wert wird durch Zugabe basisch reagierender Reagenzien zu der üblicherweise schwach alkalisch bis schwach sauer reagierenden Katalysatorlösung eingestellt. Geeignet hierfür sind Alkalihydroxide, Alkalicarbonate, ferner Ammoniak oder Amine. Falls die Alkalinität der Lösung den einzuhaltenden pH-Bereich übersteigt, setzt man ihr anorganische Säuren wie Phosphorsäure oder Schwefelsäure, saure Salze mehrwertiger anorganischer Säuren wie Alkalihydrogensulfat oder wasserlösliche organische Säuren, z.B. Ameisensäure, Essigsäure, Propionsäure zu, bis der gewünschte Wert erreicht ist. Bewährt hat es sich auch, Pufferlösungen zu verwenden, die eine präzise Einstellung des pH-Bereiches erlauben und seine Konstanz über lange Reaktionsperioden gewährleisten. Entsprechend den geforderten pH-Werten eignen sich als Puffergemische z.B. Borax-HCl, Borax-NaOH, NaHCO₃-NaOH, H₃BO₃/KCl-NaOH.

Die Wirksamkeit des in der alkalisch reagierenden Lösung vorliegenden Katalysators ist nicht beeinträchtigt, obgleich nach Auffassung der Fachwelt für die katalytische Aktivität das Vorliegen von Rhodiumhydridocarbonylen maßgebend ist. Auch auf Geschwindigkeit und Ausmaß der Umsetzung des Butadiens hat der pH-Wert keinen Einfluß. Die Gesamtmenge entstandener C₅- und C₁₀-Aldehyde bleibt etwa gleich.

Lediglich das Verhältnis von Primärprodukt, C₅-Aldehyde und Sekundärprodukt, C₁₀-Aldehyde, verschiebt sich mit steigendem pH-Wert zugunsten der C₁₀-Aldehyde. Jedoch geht bei Verwendung von Katalysatorlösungen, deren pH-Wert 11 und mehr beträgt, die Bildung des Sekundärproduktes deutlich zurück.

Die Katalysatorlösung kann dem Reaktionssystem präformiert zugesetzt werden. Mit gleich gutem Erfolg läßt sie sich aber auch aus Rhodium oder mindestens einer Rhodiumverbindung und der wäßrigen Lösung mindestens eines wasserlöslichen organischen Phosphins unter Reaktionsbedingungen im Reaktionsgemisch herstellen. Außer metallischem Rhodium in feinverteilter Form kann man als Rhodiumquelle wasserlösliche Rhodiumsalze wie Rhodiumchlorid, Rhodiumsulfat, Rhodiumacetat oder auch in Wasser schwer oder unlösliche Verbindungen wie Rhodium-2-ethylhexanoat oder Rhodiumoxide einsetzten. Sowohl Rhodium und Rhodiumverbindungen als auch die wasserlöslichen Phosphine können als reine Stoffe oder als Gemische aus zwei oder mehr Komponenten verwendet werden.

Die Rhodiumkonzentration in der wäßrigen Katalysatorlösung beträgt 20 bis 2000 Gew.-ppm, vorzugsweise 50 bis 700 Gew.-ppm, bezogen auf die wäßrige Katalysatorlösung.

Je mol Rhodium setzt man 4 bis 100 mol Phosphor in Form wasserlöslicher Phosphine ein. Phosphin/Rhodium-Verhältnisse oberhalb 15 : 1 drängen die Bildung von verzweigtem Primärprodukt zurück und begünstigen die Entstehung von Dialdehyd. Das Phosphin/Rhodium-Verhältnis hat auch Einfluß auf die Bildung von C₁₀-Aldehyden. Unterhalb eines molaren Verhältnisses P : Rh = 30 : 1 werden die Produkte der Aldolkondensation nur in geringem Umfang gebildet. Die Gesamtmenge aus unverzweigtem Monoaldehyd und dessen intermolekularem Aldolkondensationsprodukt wird durch das P/Rh-Verhältnis kaum beeinflußt.

Die Hydroformylierung des Butadiens-1,3 erfolgt bei Temperaturen zwischen 60 und 150°C, vorzugsweise 90 und 120°C. Höhere Temperaturen innerhalb des genannten Bereiches begünstigen die Dialdehydbildung. Oberhalb etwa 120°C nimmt die Entstehung von C₅-Aldehyden und ihrer intermolekularen Aldolkondensationsprodukte ab.

Bevorzugt wird die Hydroformylierung von Butadien-1,3 bei Drücken von 1 bis 10 MPa durchgeführt. Niedriger Druck innerhalb der genannten Bereiche führt bevorzugt zur Bildung von C₅-Aldehyden, beeinträchtigt jedoch den Umsatz. Drücke oberhalb 10 MPa fördern die Aldolkondensation.

Der Butadienumsatz je Zeiteinheit und die Kondensation der C₅-Aldehyde zu C₁₀-Aldehyden wird deutlich erhöht, wenn man der wäßrigen alkalischen Katalysatorlösung ein Phasentransferreagenz (Lösungsvermittler) zusetzt. Es verändert die physikalischen Eigenschaften der Grenzflächen zwischen den beiden flüssigen Phasen und erleichtert den Übergang des organischen Reaktanten in die wäßrige Katalysatorphase.

Als Lösungsvermittler sind Verbindungen bekannt, deren hydrophile Gruppen ionisch (anionisch oder kationisch) oder nichtionisch sind. Zu den anionenaktiven Verbindungen gehören Natrium-, Kalium oder Ammoniumsalze von Carbonsäuren mit 8 bis 20 Kohlenstoffatomen, insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagentien können in wäßriger Lösung nicht in Ionen dissoziieren. Zu ihnen zählen Alkylpolyethylenglykole, Alkylphenylpolyethylenglykole, Fettsäurealkylolamide und Trialkylaminoxide. Schließlich finden auch Ampholyte wie Aminocarbonsäuren, Betaine und Sulfobetaine als Lösungsvermittler Anwendung.

Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 5 Kohlenstoffatomen bedeuten und E für ein Anion, insbesondere für Sulfat, Tetrafluorborat, Acetat, Methosulfat, Benzolsulfonat, Alkylbenzolsulfonat, Toluolsulfonat, Lactat oder Citrat steht.

Das bei der Hydroformylierung anfallende Reaktionsgemisch wird durch einfache Phasentrennung vom Katalysator geschieden. Die wäßrige Katalysatorlösung kann gegebenenfalls nach Regenerierung und Einstellung der Rhodium- und/oder Phosphinkonzentration erneut eingesetzt werden. Die organische Phase wird destillativ in die Bestandteile zerlegt, die als solche verwendet oder weiterverarbeitet werden. Die C₁₀-Aldehyd-Fraktion kann unmittelbar zu 2-Propylheptanol hydriert werden. Die C₅-Aldehyde werden als Gemisch, vorzugsweise in Aldole überführt. Die Umsetzung erfolgt auf konventionellem Wege unter der Einwirkung basischer Katalysatoren. Als Katalysatoren finden Alkalicarbonate oder Alkalihydroxide, insbesondere Verbindungen des Natriums oder Kaliums und Amine, vorzugsweise tertiäre Amine, wie Triethylamin, Tri-n-propylamin, Tri-n-butylamin, Anwendung. Man arbeitet bei Temperaturen von 60 bis 160°C, insbesondere 80 bis 130°C und bei Normaldruck oder bei bis etwa 1 MPa erhöhtem Druck. Die Reaktionszeit beträgt wenige Minuten bis zu mehreren Stunden und ist insbesondere abhängig von Katalysatortyp und Reaktionstemperatur. Aufgrund ihrer höheren Reaktionsgeschwindigkeit dimerisieren vornehmlich n-C₅-Aldehyde mit sich selbst oder mit isomeren C₅-Aldehyden zu C₁₀-Aldehyden, eine Kondensation der verzweigten C₅-Aldehyde untereinander tritt dagegen völlig in den Hintergrund. Die isomeren C₁₀-Aldehyde werden zu den entsprechenden gesättigten Alkoholen hydriert. Geeignet für diese Reaktion sind Hydrierkatalysatoren auf Basis von Nickel, Chrom oder Kupfer. Üblicherweise liegt die Hydriertemperatur zwischen 100 und 180°C und der Druck zwischen 1 und 10 MPa. Zur Reinigung wird das Gemisch isomerer C₁₀-Alkohole destilliert. Unabhängig von ihrer Herkunft, unmittelbar aus der Hydroformylierungsstufe oder aus der Aldolkondensation der C₅-Aldehyde, eignen sie sich vorzüglich als Alkoholkomponente in Phthalsäureestern, die als Weichmacher verwendet werden. Die Herstellung der Phthalsäureester ist bekannt [vgl. Ullmann, Encyclopädie der Technischen Chemie (1979), Bd. 18, Seite 536 ff]. Zweckmäßig setzt man Phthalsäureanhydrid mit dem Decylalkoholgemisch im Molverhältnis 1 : 2 in einer Stufe um. Die Reaktionsgeschwindigkeit kann durch Katalysatoren und/oder durch Steigerung der Reaktionstemperatur erhöht werden. Um das Gleichgewicht in Richtung der Esterbildung zu verschieben, ist es erforderlich, das gebildete Wasser aus dem Reaktionsgemisch zu entfernen.

Die nachfolgenden Beispiele erläutern die Erfindung, sie ist jedoch nicht auf die beschriebenen Ausführungsformen beschränkt.

Die Versuchsdurchführung erfolgt in allen Beispielen in derselben Weise. In einem 125 ml Edelstahlautoklaven werden in Argon-Atomosphäre 40 g einer wäßrigen Katalysatorlösung vorgelegt, die 16,4 mg Rhodium (in Form von Rhodium-2-ethylhexanoat oder Rhodiumsulfat entsprechend 410 ppm Rh) und wechselnde Mengen Tris(m-sulfonatophenyl)phosphin-trinatriumsalz enthält und deren pH-Wert, wenn nicht anders angegeben, jeweils durch Zusatz von Natriumcarbonat oder Schwefelsäure eingestellt wird. Man behandelt die Katalysatorlösung bei 110°C und 10 MPa Druck 90 min mit Synthesegas (Volumverhältnis CO/H₂ = 1 : 1). Zu dem präformierten Katalysator gibt man jeweils 20 g Butadien-1,3, stellt mit Synthesegas den gewünschten Reaktionsdruck ein und läßt 12 h reagieren. Nach Beendigung der Umsetzung werden Katalysator und Produktphase getrennt. Die Katalysatorphase wird dreimal mit je 15 ml Ether extrahiert. Produktphase und Ether-Extrakt werden vereinigt, mit Natriumsulfat getrocknet und bei 140°C und 20 MPa Druck 12 h in Gegenwart eines Pt-Aktivkohle-Katalysators (10 Gew.-% Pt) hydriert. Das Hydrierprodukt wird gaschromatographisch analysiert.

In den Beispielen werden folgende Abkürzungen verwendet:
- n-Pentanol: n-P
- 2-Methylbutanol: 2-MB
- Cyclopentanmethanol: CPM
- 2-Propylheptanol: PH

### Beispiel 1

Reaktionsbedingungen: 120°C; 20 MPa; wäßrige/organische Phase (i.Vol.) = 2 : 1; CO/H₂ (i.Vol.) = 1 : 1; pH-Wert der wäßrigen Phase: 9.

| Versuch | P:Rh (in mol) | n-P (Gew.-%) | 2-MB (Gew.-%) | CPM (Gew.-%) | PH (Gew.-%) | (n-P+PH) (Gew.-%) |
|---|---|---|---|---|---|---|
| 1/1 | 15:1 | 45 | 4 | 7 | 9 | 54 |
| 1/2 | 30:1 | 48 | 5 | 9 | 10 | 58 |
| 1/3 | 40:1 | 48 | 4 | 8 | 13 | 61 |
| 1/4 | 60:1 | 38 | 4 | 7 | 20 | 58 |
| 1/5 | 80:1 | 42 | 3 | 8 | 18 | 60 |

### Beispiel 2

Reaktionsbedingungen: 120°C; 20 MPa; P/Rh (i.mol) = 60 : 1; wäßrige/organische Phase (i.Vol.) = 2 : 1; CO/H₂ (i.Vol.) = 1 : 1.

| Versuch | pH | n-P (Gew.-%) | 2-MB (Gew.-%) | CPM (Gew.-%) | PH (Gew.-%) | (n-P+PH) (Gew.-%) |
|---|---|---|---|---|---|---|
| 2/1 | 3 | 61 | 4 | 8 | 7 | 68 |
| 2/2 | 7 | 47 | 3 | 9 | 8 | 55 |
| 2/3 | 9 | 36 | 3 | 8 | 23 | 60 |
| 2/4 | 11 | 55 | 4 | 8 | 11 | 66 |

### Beispiel 3

Reaktionsbedingungen: 20 MPa; P/Rh (i.mol) = 60 : 1; wäßrige/organische Phase (i.Vol.) = 2 : 1; CO/H₂ (i.Vol.) = 1 : 1; pH-Wert der wäßrigen Phase: 9.

| Versuch | Temp. (°C) | n-P (Gew.-%) | 2-MB (Gew.-%) | CPM (Gew.-%) | PH (Gew.-%) | (n-P+PH) (Gew.-%) |
|---|---|---|---|---|---|---|
| 3/1 | 100 | 31 | 2 | 5 | 27 | 58 |
| 3/2 | 120 | 36 | 3 | 8 | 23 | 59 |
| 3/3 | 140 | 29 | 3 | 8 | 7 | 36 |

### Beispiel 4

Reaktionsbedingungen: 120°C; P/Rh (i.mol) = 60 : 1; wäßrige/organische Phase (i.Vol.) = 2 : 1; pH-Wert der wäßrigen Phase: 9.

| Versuch | Druck (MPa) | n-P (Gew.-%) | 2-MB (Gew.-%) | CPM (Gew.-%) | PH (Gew.-%) | (n-P+PH) (Gew.-%) |
|---|---|---|---|---|---|---|
| 4/1 | 4,0 | 57 | 2 | 12 | 8 | 65 |
| 4/2 | 6,0 | 67 | 4 | 10 | 10 | 77 |
| 4/3 | 8,0 | 55 | 3 | 9 | 13 | 68 |
| 4/4 | 12,0 | 49 | 4 | 8 | 18 | 67 |
| 4/5 | 16,0 | 28 | 2 | 6 | 23 | 51 |
| 4/6 | 20,0 | 36 | 3 | 8 | 23 | 59 |
| 4/7 | 26,0 | 52 | 3 | 8 | 15 | 67 |

### Beispiel 5

Reaktionsbedingungen: 100°C; 5,5 MPa; P/Rh (i.mol) = 60 : 1; wäßrige/organische Phase (i.Vol.) = 2 : 1; CO/H₂ (i.Vol.) = 1 : 1.

| Versuch | pH | n-P (Gew.-%) | 2-MB (Gew.-%) | CPM (Gew.-%) | PH (Gew.-%) | (n-P+PH) (Gew.-%) |
|---|---|---|---|---|---|---|
| 5/1 | 9 | 33 | 2 | 4 | 25 | 58 |
| 5/2 | 9 | 26 | 2 | 4 | 32 | 58 |
| 5/3 | 11 | 49 | 2 | 1 | 19 | 68 |
| 5/4 | 11 | 53 | 2 | 1 | 22 | 75 |

Die Ergebnisse dieser Versuche zeigen, daß die Selektivität der Reaktion für das Aldolisierungsprodukt 2-Propylheptenal befriedigend hoch auch bei Drücken um 5 MPa ist. Die Selektivität für das Aldolkondensationsprodukt sinkt beim Übergang vom schwach alkalischen in den stärker alkalischen Bereich.

### Beispiel 6

Reaktionsbedingungen: 100°C; 5,5 MPa; P/Rh (i.mol) = 60 : 1; wäßrige/organische Phase (i.Vol.) = 2 : 1; CO/H₂ (i.Vol.) = 1 : 1; pH-Wert der wäßrigen Phase: 9 (in den Versuchen 6/2 bis 6/4 mit Hilfe eines H₃BO₃/KCl-NaOH-Puffers eingestellt); DTAB: Dodecyltrimethylammoniumbromid.

| Versuch | Tensid | Puffer | n-P (Gew.-%) | 2-MB (Gew.-%) | CPM (Gew.-%) | PH (Gew.-%) | (n-P+PH) (Gew.-%) |
|---|---|---|---|---|---|---|---|
| 6/1 | DTAB | - | 24 | 2 | 8 | 20 | 44 |
| 6/2 | - | + | 27 | 2 | 4 | 19 | 46 |
| 6/3 | - | + | 32 | 2 | 5 | 18 | 50 |
| 6/4 | DTAB | + | 22 | 3 | 7 | 29 | 51 |

Die Ergebnisse zeigen, daß die Selektivität der Reaktion hinsichtlich der Bildung von linearem C₅-Aldehyd und dessen Aldolkondensationsfolgeprodukt sinkt, wenn unter Zusatz von Tensid oder Puffergemischen gearbeitet wird.

Die Zunahmen des Umsatzes/Zeiteinheit bei Zusatz eines Tensids zur Katalysatorlösung zeigen die nachstehenden Versuche

| Reaktionszeit / h | Druckabnahme / MPa | |
|---|---|---|
| | ohne Tensid | unter Tensidzusatz |
| 1 | 0.7 | 2.0 |
| 2 | 1.3 | 3.1 |
| 4 | 2.8 | 4.9 |
| 8 | 4.3 | 6.0 |
| 12 | 5.1 | 6.3 |

### Beispiel 7 (Vergleich)

Dieses Vergleichsbeispiel zeigt, daß sich Buten-1 bei der Hydroformylierung unter den erfindungsgemäßen Bedingungen völlig anders verhält als Butadien-1,3. Das hydrierte Reaktionsprodukt besteht im wesentlichen aus n-Pentanol und enthält 2-Propylheptanol nur in ganz untergeordneten Mengen.

Reaktionsbedingungen: 100°C; 5,5 MPa; P/Rh (i.mol) = 60 : 1; wäßrige/organische Phase (i.Vol.) = 2 : 1; CO/H₂ (i.Vol.) = 1 : 1; pH-Wert der wäßrigen Phase : 9.

| Versuch | n-P (Gew.-%) | 2-MB (Gew.-%) | PH (Gew.-%) |
|---|---|---|---|
| 7/1 | 91 | 4 | 1 |
| 7/2 | 93 | 4 | 0,4 |

## Patentansprüche

1. Verfahren zur Hydroformylierung von Butadien-1,3 bei Temperaturen von 60 bis 150°C und Drücken von 1 bis 20 MPa in Gegenwart einer wäßrigen Lösung als Katalysator, die mindestens eine wasserlösliche Rhodiumverbindung und mindestens ein wasserlösliches, organisches Phosphin enthält, dadurch gekennzeichnet, daß die Umsetzung des Butadiens bei einem pH-Wert in der wäßrigen Lösung von 8,0 bis 11,0 erfolgt.

2. Verfahren nach Anspruch 1, dadurch gekennzeichnet, daß der pH-Wert in der wäßrigen Lösung 8,5 bis 9,5 beträgt.

3. Verfahren nach Anspruch 1 oder 2, dadurch gekennzeichnet, daß die Einstellung des pH-Wertes mit Hilfe eines Puffergemisches erfolgt.

4. Verfahren nach einem oder mehreren der Ansprüche 1 bis 3, dadurch gekennzeichnet, daß die Umsetzung bei Temperaturen von 90 bis 120°C erfolgt.

5. Verfahren nach einem oder mehreren der Ansprüche 1 bis 4, dadurch gekennzeichnet, daß die Umsetzung bei Drücken von 1 bis 10 MPa durchgeführt wird.

6. Verfahren nach einem oder mehreren der Ansprüche 1 bis 5, dadurch gekennzeichnet, daß die Rhodiumkonzentration in der wäßrigen Katalysatorlösung 20 bis 2000 Gew.-ppm, vorzugsweise 50 bis 700 Gew.-ppm, bezogen auf die wäßrige Katalysatorlösung, beträgt.

7. Verfahren nach einem oder mehreren der Ansprüche 1 bis 6, dadurch gekennzeichnet, daß in der wäßrigen Lösungen je mol Rhodium 4 bis 100 mol Phosphor, vorzugsweise 10 bis 60 mol Phosphor, vorhanden sind.

8. Verfahren nach einem oder mehreren der Ansprüche 1 bis 7, dadurch gekennzeichnet, daß als wasserlösliche Phosphine mindestens eine Sulfonsäuregruppe oder mindestens eine Carboxylgruppe enthaltende Triarylphosphine, Trialkylphosphine, Tri(alkyl-aryl)phosphine, Alkylendiphosphine oder Aryldiphosphine verwendet werden.

9. Verfahren nach einem oder mehreren der Ansprüche 1 bis 8, dadurch gekennzeichnet, daß die wäßrige Lösung einen Lösungsvermittler enthält.

## Claims

1. A process for the hydroformylation of 1,3-butadiene at temperatures of 60 to 150°C and pressures of 1 to 20 MPa in the presence of an aqueous solution as catalyst which contains at least one water-soluble rhodium compound and at least one water-soluble organic phosphine, which comprises reacting the butadiene at a pH in the aqueous solution of 8.0 to 11.0.

2. The process as claimed in claim 1, wherein the pH in the aqueous solution is 8.5 to 9.5.

3. The process as claimed in claim 1 or 2, wherein the pH is adjusted with the aid of a buffer mixture.

4. The process as claimed in one or more of claims 1 to 3, wherein the reaction proceeds at temperatures of 90 to 120°C.

5. The process as claimed in one or more of claims 1 to 4, wherein the reaction is carried out at pressures of 1 to 10 MPa.

6. The process as claimed in one or more of claims 1 to 5, wherein the rhodium concentration in the aqueous catalyst solution is 20 to 2000 ppm by weight, preferably 50 to 700 ppm by weight, based on the aqueous catalyst solution.

7. The process as claimed in one or more of claims 1 to 6, wherein, per mole of rhodium, 4 to 100 mol of phosphorus, preferably 10 to 60 mol of phosphorus, are present in the aqueous solutions.

8. The process as claimed in one or more of claims 1 to 7, wherein the water-soluble phosphines used are triarylphosphines, trialkylphosphines, tri(alkylaryl)phosphines, alkylenediphosphines or aryldiphosphines containing at least one sulfonic acid group or at least one carboxyl group.

9. The process as claimed in one or more of claims 1 to 8, wherein the aqueous solution contains a solubilizer.

## Revendications

1. Procédé pour l'hydroformylation de 1,3-butadiène à des températures de 60 à 150°C et à des pressions de 1 à 20 MPa en présence d'une solution aqueuse comme catalyseur, qui contient au moins un composé du rhodium soluble dans l'eau et au moins une phosphine organique soluble dans l'eau, caractérisé en ce qu'on réalise la transformation du butadiène à une valeur du pH dans la solution aqueuse de 8,0 à 11,0.

2. Procédé selon la revendication 1, caractérisé en ce que la valeur du pH dans la solution aqueuse est de 8,5 à 9,5.

3. Procédé selon la revendication 1 à 2, caractérisé en ce que l'ajustement de la valeur du pH se fait à l'aide d'un mélange tampon.

4. Procédé selon l'une ou plusieurs quelconques des revendications 1 ou 3, caractérisé en ce que la transformation se fait à des températures de 90 à 120°C.

5. Procédé selon l'une ou plusieurs quelconques des revendications 1 à 4, caractérisé en ce qu'on réalise la transformation à des pressions de 1 à 10 MPa.

6. Procédé selon l'une ou plusieurs quelconques des revendications 1 à 5, caractérisé en ce que la concentration en rhodium dans la solution aqueuse de catalyseur est de 20 à 2 000 ppm en poids, de préférence de 50 à 700 ppm en poids, rapportées à la solution aqueuse de catalyseur.

7. Procédé selon l'une ou plusieurs quelconques des revendications 1 à 6, caractérisé en ce que de 4 à 100 moles de phosphore, de préférence de 10 à 60 moles de phosphore, sont présentes pour chaque mole de rhodium dans la solution aqueuse.

8. Procédé selon l'une ou plusieurs quelconques des revendications 1 à 7, caractérisé en ce qu'on utilise comme phosphine soluble dans l'eau une triarylphosphine, une trialkylphosphine, une tri(alkyl-aryl)phosphine, une alkylendiphosphine ou une aryldiphosphine contenant au moins un groupe d'acide sulfonique ou au moins un groupe carboxyle.

9. Procédé selon l'une ou plusieurs quelconques des revendications 1 à 8, caractérisé en ce que la solution aqueuse contient un agent de dissolution.
